# EUROPEAN PATENT APPLICATION

(11) **EP 3 608 402 A1**
(43) Date of publication of application: **12.02.2020**
(21) Application number: 18188107.9
(22) Date of filing: 08.08.2018
(51) Int. Cl.: C12N 9/16, A23K 10/00, A23K 20/189

(54) **OPTIMIZED 3-PHYTASE AND ITS USE IN FOOD OR FEED PRODUCTION**

(71) Applicant: Clariant Produkte (Deutschland) GmbH, 65929 Frankfurt am Main (DE)
(72) Inventor: JOCHENS, Helge, 82065 Baierbrunn (DE); WALLRAPP, Frank, 82131 Gauting (DE); HOESL, Michael, 80469 München (DE); EISELE, Thomas, 82223 Eichenau (DE); ROECHER, Lutz, 81541 München (DE); GEBHARDT-WEIDL, Gabi, 84051 Essenbach (DE); GRAEBER, Martin, 82131 Stockdorf (DE); STUERMER, Werner, 82266 Inning (DE); CLAREN, Jörg, 82131 Stockdorf (DE); O'CONNELL, Timothy, 86899 Landsberg am Lech (DE); SCHATTE, Martin, 81479 München (DE); REISINGER, Christoph, 82131 Gauting (DE)
(74) Representative: Graser, Konstanze

(57) **Abstract**

The present invention refers to an optimized 3-phytase which is a bio-efficacious phytase mutant having sequence identity of at least 95 % around 3-phytase (SEQ ID NO. 1) comprising a sequence of SEQ ID NO.2 and one or more mutations. The invention is further directed to the use of such optimized 3-phytase in the production of a food or feed product.

## Description

The present invention refers to an optimized 3-phytase which is a bio-efficacious phytase mutant having sequence identity of at least 95 % around 3-phytase (SEQ ID NO. 1) comprising a sequence of SEQ ID NO.2 and one or more mutations. The invention is further directed to the use of such optimized 3-phytase in the production of a food or feed product.

### Technical background

Phytases are enzymes which remove phosphate from organophosphate compounds such as phytate and phytic acid, respectively. Many types of phytases are known in the art and some of them are already used as an additive to animal feed products in order to improve metabolization and the utilization of phytate naturally present within animal feed stuff such as corn, wheat and soybeans. The classification of the phytases depends on the starting position of cleavage of phosphate from the phytate molecule in the first place. 3-phytases (E.C. 3.1.3.8, also referred to as: 1 -phytase; myo-inositol-hexakisphosphate 3-phosphohydrolase; phytate 1 -phosphatase or phytate 3 - phosphatase) start with the phosphate cleavage at position 3 of the phytate molecule. 6-phytases start with the phosphate cleavage at position 6. Depending on the type of phytase, further phosphate cleavage of other positions occurs. 3-phytases for example are able to cleave all phosphate groups from phytate.

The addition of a phytase to food or feed enables better metabolization of the natural phytate content of the food or feed, as phosphate is an important element of a human's or an animal's diet, but also leads to a decrease of remaining phosphor in the excrements. High phosphorous contents in excrements usually leads to severe limitations of its use as manure as many countries set strict limitations.

In order to overcome these drawbacks of an insufficient natural phytate metabolization and a costly artificial phosphorous enrichment of food or feed which is also known to particularly increase remaining phosphorous content in manure, phytases have already been introduced in food or feed products to increase phytate metabolization in the stomach and digestive tract.

Examples for such phytases which are currently commercially available originate from *Escherichia coli, Citrobacter braakii, Buttiauxella species and Aspergillus niger.*

Each of these phytase products has limitations in at least specific activity, dosage requirements, reaction time and accumulation of inhibiting intermediate compounds. First of its kind products contained fungal 3-phytases which require high enzyme dosing in order to achieve the desired effect. Currently available, commercial feed products try to overcome this effect by using 6-phytases of bacterial origin. A severe drawback of these 6-phytases is, however, an accumulation of the intermediate compound inositol-tetraphosphate which severely limits efficient phytate metabolization. In addition, phytate and its intermediates, particularly inositol-tetraphosphate, form complexes with divalent ions such as Ca²⁺, Zn²⁺ and Mg²⁺. Further, many of the phytase products known within the art require the use of a high amount of phytase to guarantee a sufficient phosphate release regarding the actual critical retention time of the food or feed in the humans or animals stomach of a maximum of 1.5 hours.

Several factors influence phytase functionality and their *in vivo* phytase enzyme activity in the digestive tract of humans or animals In pigs for example, feed phytase is mainly active in the stomach and upper part of the small intestine, and added phytase activity is not recovered in the ileum. In poultry for example, feed phytase activities are mainly found in the upper part of the digestive tract, including the crop, proventriculus and gizzard. For fish with a stomach for example, phytase activities are mainly in the stomach. Many factors can influence the efficiency of food or feed phytase in the gastrointestinal tract, and they can be divided into three main groups: (i) phytase related; (ii) dietary related and (iii) subject related. Phytase-related factors include type of phytase (e.g. 3- or 6-phytase; bacterial or fungal phytase origin), the pH optimum and the resistance of phytase to endogenous protease. Dietary-related factors are mainly associated with dietary phytate content, food or feed ingredient composition and food or feed processing, and total P, Ca and Na content. Subject-related factors include species, gender and age of subjects (Yueming Dersjant-Li et al., J. Sci. Food Agric., 2015 Mar 30; 95(5): 878-896).

Food and feed, respectively, is generally produced at high temperatures, e.g., to reduce contamination, and in consequence phytases used in food and/or feed production have to be thermostable.

Therefore, there is a need for a phytase that works over a wide range of pH values and thus, is active in the stomach as well as the upper intestine and that is at the same time stable at high temperature.

The present invention fills this gap and provides 3-phytase mutants such as bacterial 3-phytase mutants comprising mutations which result in unexpected and surprising temperature and pH stability, wherein the 3-phytase is active and highly efficient in phosphate cleavage.

### Summary

The present invention refers to a bio-efficacious phytase mutant such as a 3-phytase mutant having sequence identity of at least 96.25 % around SEQ ID NO. 1, wherein the mutant comprises a sequence of SEQ ID NO.2 and a mutation selected from the group consisting of D149N, S189D, K190N, K191N, R210Q, L211E, L211M, K25E, K25A, G26N, G26S, N193H, N193K, N193S, N193R, D1E, P3T, G26K, K29A, T30S, T30A, T30K, D36N, Q45N, Q45R, V58E, S75C, Q76R, P107S, A112G, D120K, T129K, Q136R, Q144L, D149N, K187E, D189N, G192R, S195R, S195Y, S195T, S195N, N204I, N204T, N204S, S214A, N216D, A230T, M237L, N243K, N248K, R255H, G257S, F264Y, A275P, D276G, K295Y, K295M, Q332D, A352S, V383M, R393H, A395T and combinations thereof.

The bio-efficacious phytase mutant of the present invention comprises for example mutations D149N, S189D, K190N and K191N, or R210Q and L211M, or K25E or K25A, and G26N or G26S, and N193S or N193R; or D149N, S189D, K190N, K191N, R210Q, L211M, K25E or K25A, and G26N or G26S, and N193S or N193R.

The bio-efficacious phytase mutant of the present invention comprises for example mutations at positions 25, 26, 193, 210 and/or 211.

The bio-efficacious phytase mutant of the present invention is active for example up to 90 °C and active for example at a pH from 3 to 5.5.

The bio-efficacious phytase mutant of the present invention is used for example for the production of a food or feed product for example for humans and/or animals.

All documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention. More specifically, all referenced documents are incorporated by reference to the same extent as if each individual document was specifically and individually indicated to be incorporated by reference.

### Description of figures

The following figures show the invention in more detail, however, the invention is not limited to these figures.

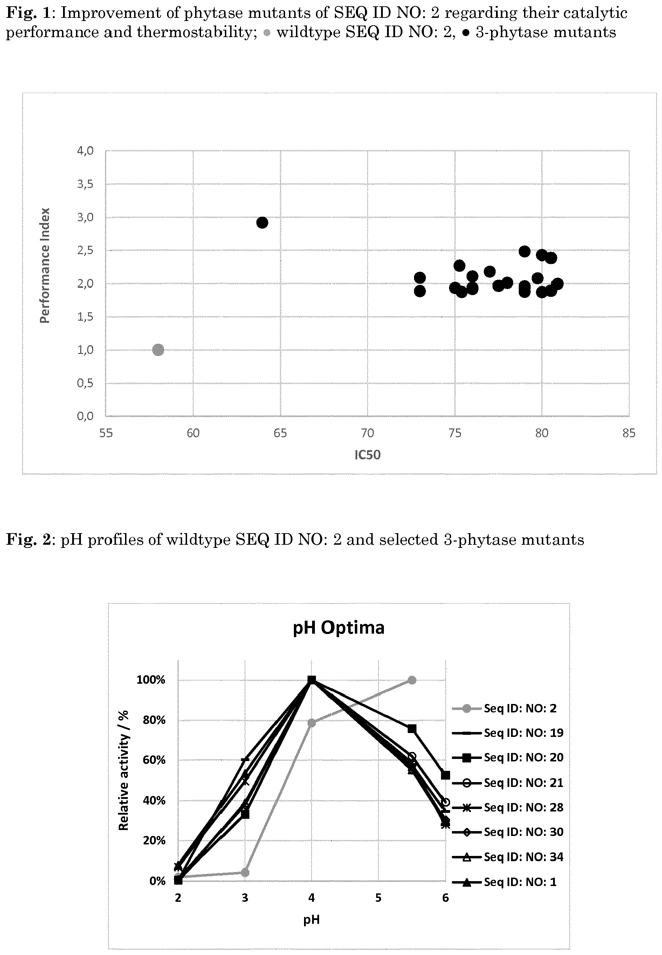

**Fig. 1****:** Improvement of 3-phytase mutants of SEQ ID NO: 2 regarding their catalytic performance in an application setup and their IC₅₀ values as a measure for thermostability; 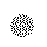 wildtype SEQ ID NO: 2, ● 3-phytase mutants of SEQ ID: NO: 47, SEQ ID: NO: 4, SEQ ID: NO: 6, SEQ ID: NO: 7, SEQ ID: NO: 8, SEQ ID: NO: 10, SEQ ID: NO: 14, SEQ ID: NO: 19, SEQ ID: NO: 20, SEQ ID: NO: 21, SEQ ID: NO: 26, SEQ ID: NO: 28, SEQ ID: NO: 29, SEQ ID: NO: 30, SEQ ID: NO: 31, SEQ ID: NO: 34, SEQ ID: NO: 1, SEQ ID: NO: 37, SEQ ID: NO: 38, SEQ ID: NO: 39, SEQ ID: NO: 40, SEQ ID: NO: 41, SEQ ID: NO: 45, and SEQ ID: NO: 46.
**Fig.2****:** pH profiles of wildtype SEQ ID NO:2 and 3-phytase mutants of SEQ ID: NO: 19, SEQ ID: NO: 20, SEQ ID: NO: 21, SEQ ID: NO: 28, SEQ ID: NO: 30, SEQ ID: NO: 34, SEQ ID: NO: 1 at pH 2 to 5.5.

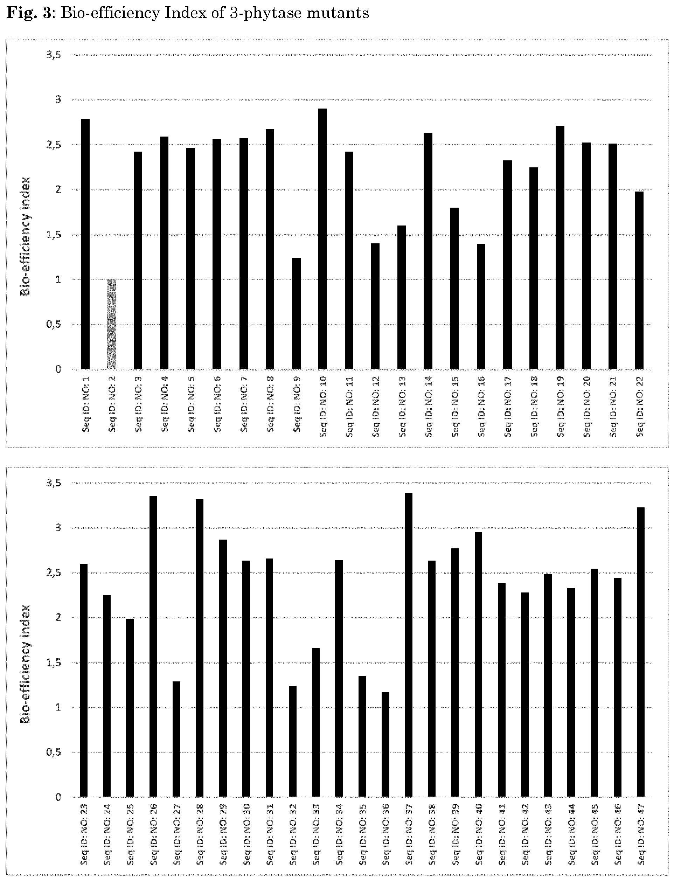

**Fig. 3****:** Bio-efficiency Index of 3-phytase mutants SEQ ID: NO: 1, SEQ ID: NO: 3, SEQ ID: NO: 4, SEQ ID: NO: 5, SEQ ID: NO: 6, SEQ ID: NO: 7, SEQ ID: NO: 8, SEQ ID: NO: 9, SEQ ID: NO: 10, SEQ ID: NO: 11, SEQ ID: NO: 12, SEQ ID: NO: 13, SEQ ID: NO: 14, SEQ ID: NO: 15, SEQ ID: NO: 16, SEQ ID: NO: 17, SEQ ID: NO: 18, SEQ ID: NO: 19, SEQ ID: NO: 20, SEQ ID: NO: 21, SEQ ID: NO: 22, SEQ ID: NO: 23, SEQ ID: NO: 24, SEQ ID: NO: 25, SEQ ID: NO: 26, SEQ ID: NO: 27, SEQ ID: NO: 28, SEQ ID: NO: 29, SEQ ID: NO: 30, SEQ ID: NO: 31, SEQ ID: NO: 32, SEQ ID: NO: 33, SEQ ID: NO: 34, SEQ ID: NO: 35, SEQ ID: NO: 36, SEQ ID: NO: 37, SEQ ID: NO: 38, SEQ ID: NO: 39, SEQ ID: NO: 40, SEQ ID: NO: 41, SEQ ID: NO: 42, SEQ ID: NO: 43, SEQ ID: NO: 44, SEQ ID: NO: 45, SEQ ID: NO: 46, SEQ ID: NO: 47 in comparison to SEQ ID NO:2.

### Detailed description

The present invention is directed to a phytase mutant, such as a 3-phytase mutant having sequence identity to SEQ ID NO: 1 of 90 % to 97 %, 91 % to 96 %, 92 % to 95 %, at least 90 %, at least 91 %, at least 92 %, at least 93 %, at least 94 %, at least 95 %, at least 96.25 % or at least 97 % comprising or consisting of the sequence of SEQ ID NO.2 and one or more, e.g., at least two, at least three, at least four, at least five, at least six, at least seven, at least eight, at least nine or at least ten mutations.

The mutation of the phytase such as the 3-phytase of the present invention is selected from the group consisting of D149N, S189D, K190N, K191N, R210Q, L211E, L211M, K25E, K25A, G26N, G26S, N193H, N193K, N193S, N193R, D1E, P3T, G26K, K29A, T30S, T30A, T30K, D36N, Q45N, Q45R, V58E, S75C, Q76R, P107S, A112G, D120K, T129K, Q136R, Q144L, D149N, K187E, D189N, G192R, S195R, S195Y, S195T, S195N, N204I, N204T, N204S, S214A, N216D, A230T, M237L, N243K, N248K, R255H, G257S, F264Y, A275P, D276G, K295Y, K295M, Q332D, A352S, V383M, R393H, A395T and combinations thereof. The combinations comprise one or more mutations.

The resulting phytase mutant is bio-efficacious, i.e., the phytase mutant is consistently active under varying conditions which are in the present invention high temperatures for example up to 90 °C and acidic to basic pH for example in the range from pH 2.5 to 5.5.

In the following, the elements of the present invention will be described in more detail. These elements are listed with specific embodiments, however, it should be understood that they may be combined in any manner and in any number to create additional embodiments. The variously described examples and embodiments should not be construed to limit the present invention to only the explicitly described embodiments. This description should be understood to support and encompass embodiments which combine the explicitly described embodiments with any number of the disclosed elements. Furthermore, any permutations and combinations of all described elements in this application should be considered disclosed by the description of the present application unless the context indicates otherwise.

Throughout this specification and the claims, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example"), provided herein is intended merely to better illustrate the invention and does not pose a limitation on the scope of the invention otherwise claimed. No language in the specification should be construed as indicating any non-claimed element essential to the practice of the invention.

The temperature for example of a food or feed product and thus, of a phytase mutant for example contained in the food or feed product varies significantly with a storage temperature for example of -18 °C, 4° C or room temperature, the temperature in a subject such as a human or animal which is for example 37 °C, and the temperature for the preparation of food or feed product for example in decontamination processes with temperatures, e.g., of up to 90 °C. The bio-efficacious phytase mutant of the present invention is stable and active from 4 °C, 10 °C, 15 °C, 20 °C, 25 °C, 28 °C, 30 °C, 35 °C, 37 °C, 40 °C, 45 °C, 50 °C, 55 °C, 60 °C, 65 °C, 70 °C, or 75 °C to 90 °C, or is stable and active up to 40 °C, 45 °C, 50 °C, 55 °C, 60 °C, 65 °C, 70 °C, 75 °C, 80 °C, 85 °C or 90 °C. In addition, a phytase passing through the intestinal tract of a human or an animal is exposed to different pH ranges for example in humans the saliva has a pH of about 6.5 to 7.5, the upper stomach has a pH of about 4.0 to 6.5, the lower stomach has a pH of about 1.5 to 4.0, the duodenum has a pH of about 7.0 to 8.5 and the small and large intestine have a pH of about 4.0 to 7.0. A highly efficient phytase mutant such as a 3-phytase mutant resulting in a high cleavage rate of phosphate from a phytate has to be active in a broad pH range. The bio-efficacious phytase mutant of the present invention is stable and active in a range from pH 2.5 to 5.5., from pH 3.0 to 5.0, from pH 3.5 to 4.5, from 3.0 to 5.5 or from pH 5.0 to 5.5 and for example at any of the previously disclosed temperatures and temperature ranges, respectively.

The activity of a phytase and the phytase mutants of the present invention is determined by measuring the phosphate release which is indicated in mmol/L phytate at a specific pH. The Performance Index, which provides information about the activity of the phytase mutant at different pH in comparison to a reference such as SEQ ID NO: 2, is defined as [(Released phosphate [mM] at pH X)ₘᵤₜₐₙₜ/ released phosphate [mM] at pH X)_{reference}] x [(released phosphate [mM] at pH Y)ₘᵤₜₐₙₜ / (released phosphate [mM] at pH Y) _{referenc}], wherein X and Y are different pH values, i.e., for example [(Released phosphate [mM] at pH 3)ₘᵤₜₐₙₜ/ released phosphate [mM] at pH 3)_{SEQ ID NO: 2}] x [(released phosphate [mM] at pH 5)ₘᵤₜₐₙₜ / (released phosphate [mM] at pH 5) _{SEQ ID NO: 2}].

The Bio-efficiency Index determines the correlation of performance and thermostability of a phytase mutant in comparison to a reference, e.g., SEQ ID NO.2, and is defined as IC₅₀₍ᵥₐᵣᵢₐₙₜ₎/ IC_{50(reference)} x [(Released phosphate [mM] at pH X)ₘᵤₜₐₙₜ/ released phosphate [mM] at pH X)_{reference}] x [(released phosphate [mM] at pH Y)ₘᵤₜₐₙₜ / (released phosphate [mM] at pH Y)_{reference}], wherein X and Y are different pH values, i.e., for example IC₅₀₍ᵥₐᵣᵢₐₙₜ₎ / IC_{50(SEQ ID NO: 2}) x [(Released phosphate [mM] at pH 3)ₘᵤₜₐₙₜ/ released phosphate [mM] at pH 3)_{SEQ ID NO: 2}] x [(released phosphate [mM] at pH 5)ₘᵤₜₐₙₜ / (released phosphate [mM] at pH 5) _{SEQ ID NO: 2}].

The term "feed" or "feed product" as used according to the present invention pertains to any product known to a person skilled in the art as suitable for feeding any kind of animal, for example mammals and birds, such as but not limited to ruminants, pigs, deer, poultry but also comprises fish or other aquatic livestock generally referred to as seafood. Included are feed products for non-ruminant animals, e.g. poultry, broilers, birds, chickens, turkeys, ducks, geese, and fowl; ruminant animals e.g. cows, cattle, horses, and sheep; pigs, swine, piglets, growing pigs, and sows; companion animals including but not limited to: cats, dogs, rodents, and rabbits; fish including but not limited to salmon, trout, tilapia, catfish and carp; and crustaceans including but not limited to shrimp and prawn.
The term "food" or "food product" as used according to the present invention pertains to any product known to a person skilled in the art as suitable for human consumption.

The feed or food product of the present invention for example contains starch. The starch-containing feed components typically include vegetable material such as cereal(s), e.g., one or more of corn (maize), wheat, barley, rye, rice, sorghum and millet, and/or tubers such as potatoes, cassava and sweet potato. The vegetable material may be milled, e.g., wet or dry milled grain, or distillers dry grain solids.

The feed or food product of the present invention for example contains protein-rich feed ingredients such as soybean (preferably soybean meal), rapeseed, palm kernel, cotton seed and sunflower.

Further, the feed or food product comprises for example at least one compound selected from the group consisting of vitamins, minerals, organic acids, probiotic components, oils, fats, pigments, growth factors and antimicrobial agents.

Optionally the feed or food product comprises at least one or more enzymes in addition to the 3-phytase mutant, for example food or feed enzymes which improve the digestibility of the feed and/or degrade intermediate products produced by the 3-phytase mutant such as inositol-tetraphosphate, e.g. another phytase such as 6-phytase, an amylase or a protease, aminopeptidase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, invertase, laccase, lipase, mannosidase, oxidase, pectinolytic enzyme, peptidogluta-minase, peroxidase, polyphenoloxidase, proteolytic enzyme, ribonuclease, transglutaminase, or xylanase. The food or feed enzyme(s) are for example derived from microorganisms such as bacteria or fungi or from plants or animals.

The feed or food product is for example in form of a granulate, compactate, extrudate or liquid form.

The phytase mutant of the present invention is administered to a subject for example in a food or feed product, or directly without a food or feed product for example in form of a tablet, powder, liquid, an extrudate or granulate such as a coated granulate, e.g. with a coating comprising a salt, e.g. at least 60 % w/w of the salt for example selected from the group consisting of NaCI, KCI, Na₂S0₄, K₂S0₄ and MgS0₄.

The phytase mutant of the present invention is used for example for the production of a food or feed product such as pelleted or extrudated mixtures of grains, corn, hay, molasses and straw for example in dry or liquid form.

As used herein "liquid form" pertains to a composition for example comprising a buffer, a stabilizer, and an anti-microbial agent and optionally at least one further enzyme, wherein the enzyme is an amylase, a cellulase, a lactase, a lipase, a protease, a catalase, a xylanase, a beta- glucanase, a mannanase, an amylase, an amidase, an epoxide hydrolase, an esterase, a phospholipase, a transaminase, an amine oxidase, a cellobiohydrolase, an ammonia lyase, or any combination thereof.
As used herein "dry form" pertains to a composition for example comprising one or more of the following components: a carrier, a buffer, a stabilizer, a binding agent, a plasticizer, an anti-microbial agent. Dry forms of a food or feed product comprise for example sucrose, sodium chloride, sorbitol, sodium citrate, potassium sorbate, sodium benzoate, sodium propionate, guar gum and/or wheat flour.

Sequences of phytase mutants of the present invention are for example the following, which were partially tested in the Examples:
**SEQ ID NO: 1** 
DTPSSGWTLEKVVEVSRHGVRPPTENNVKTIQEGTNRQWPTWLTQYGELTGHGYAA
AVLKGHYEGQYLRQHGLLCQACPAPGDVFVWASPLQRTRETAMALMDGVFSGCGVA
IQGPEDEDNDPLFHAEKAGVTLDQQQVKADLQNAMQNKSAAQIQAQWQPAIDRLKQ
AVCLPDKPCPAFSTPWEVKEDNNGRIYLHGPEVLANIAETIQMSYSNNNPLSEVAFG
HAKTAAEVAALMPLLTAKYDFTNDLPYVARRGASVLMNQIALALNTEHQADAPPDA
KWLLYVAHDTNIAYLRTMLGFTWQMGDYPRGNIPPAGSLIFERWRNQQSGQRVVRIY
 
FQAQSLDQIRGLAALDDSHPPLRSEFSMPDCQKTDIGTLCPYDAVMKRLNDAVDRTA
LLPVHYTPHHHHHH
**SEQ ID NO: 2** 
DTPSSGWTLEKVVEVSRHGVRPPTKGNVKTIQEGTDRQWPTWLTQYGELTGHGYAA
AVLKGHYEGQYLRQHGLLSQACPAPGDVFVWASPLQRTRETAMALMDGVFPGCGVA
IQGPEDEDNDPLFHAETAGVTLDQQQVKADLQNAMQDKSAAQIQAQWQPAIDRLKQ
AVCLPDKPCPAFSTPWEVKESKKGNISLHGPEVLANIAETIRLSYSNNNPLSEVAFGH
AKTAAEVAALMPLLTANYDFTNDLPYVARRGASVLMNQIALALNTEHQADAPPDAK
WLLYVAHDTNIAKLRTMLGFTWQMGDYPRGNIPPAGSLIFERWRNQQSGQRVVRIYF
QAQSLDQIRGLAALDDSHPPLRSEFSMPDCQKTDIGTLCPYDAVMKRLNDAVDRTAL
LPVHYTPHHHHHH
**SEQ ID NO: 3** 
DTPSSGWTLEKVVEVSRHGVRPPTENNVKTIQEGTNRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSISLHGPEVLAIIAETIQMSYSNNNPLSEVAFG
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAMMKRLNDAVDRT
ALLPVHYTPHHHHHH
**SEQ ID NO: 4** 
DTPSSGWTLEKVVEVSRHGVRPPTESNVKTIQEGTNRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSISLHGPEVLAIIAETIQMSYSNNNPLSEVAFG
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 5** 
DTPSSGWTLEKVVEVSRHGVRPPTENNVKTIQEGTNRQWPTWLTQYGELTGHGYAA
AVLKGHYEGQYLRQHGLLCQACPAPGDVFVWASPLQRTRETAMALMDGVFSGCGVA
IQGPEDEDNDPLFHAETAGVTLDQQQVKADLQNAMQNKSAAQIQAQWQPAIDRLKQ
AVCLPDKPCPAFSTPWEVKEDNNGSISLHGPEVLAIIAETIQMSYSNNNPLSEVAFGH
AKTAAEVAALMPLLTANYDFTNDLPYVARRGASVLMNQIALALNTEHQADAPPDAK
WLLYVAHDTNIAKLRTMLGFTWQMGDYPRGNIPPAGSLIFERWRNQQSGQRVVRIYF
QAQSLDQIRGLAALDDSHPPLRSEFSMPDCQKTDIGTLCPYDAVMKRLNDAVDRTAL
LPVHYTPHHHHHH
**SEQ ID NO:** 6 
DTPSSGWTLEKVVEVSRHGVRPPTESNVKTIQEGTDRQWPTWLTRYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSISLHGPEVLAIIAETIQMSYSNNNPLSEVAFG
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 7** 
DTPSSGWTLEKVVEVSRHGVRPPTESNVKTIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLLNAMQNKSAAQIQAQWQPAIDRLK
QAVCLPDKPCPAFSTPWEVKEDNNGSISLHGPEVLAIIAETIQMSYSNNNPLSEVAFG
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 8** 
DTPSSGWTLEKVVEVSRHGVRPPTESNVKTIQEGTNRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSISLHGPEVLAIIAETIQMSYSNNNPLSEVAFG
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDHT
ALLPVHYTPHHHHHH
**SEQ ID NO: 9** 
DTPSSGWTLEKVVEVSRHGVRPPTESNVKTIQEGTDRQWPTWLTQYGELTGHGYAA
AVLKGHYEGQYLRQHGLLSQACPAPGDVFVWASPLQRTRETAMALMDGVFPGCGVA
IQGPEDEDNDPLFHAEKAGVTLDQQQVKADLQNAMQNKSAAQIQAQWQPAIDRLKQ
AVCLPDKPCPAFSTPWEVKEDNNGSISLHGPEVLAIIAETIQMSYSNNNPLSEVAFGH
AKTAAEVAALMPLLTANYDFTNDLPYVARRGASVLMNQIALALNTEHQADAPPDAK
WLLYVAHDTNIAKLRTMLGFTWQMGDYPRGNIPPAGSLIFERWRNQQSGQRVVRIYF
QAQSLDQIRGLAALDDSHPPLRSEFSMPDCQKTDIGTLCPYDAVMKRLNDAVDRTAL
LPVHYTPHHHHHH
**SEQ ID NO: 10** 
DTPSSGWTLEKVVEVSRHGVRPPTESNVKTIQEGTDRQWPTWLTQYGELTGHGYAA
AVLKGHYEGQYLRQHGLLCRACPAPGDVFVWASPLQRTRETAMALMDGVFPGCGVA
IQGPEDEDNDPLFHAETAGVTLDRQQVKADLQNAMQNKSAAQIQAQWQPAIDRLKQ
AVCLPDKPCPAFSTPWEVKEDNNGSISLHGPEVLATIAETIQMSYSNNNPLSEVAFGH
 
AKTAAEVAALMPLLTANYDYTNDLPYVARRGASVLMNQIALALNTEHQADAPPDAK
WLLYVAHDTNIAKLRTMLGFTWQMGDYPRGNIPPAGSLIFERWRNQQSGQRVVRIYF
QAQSLDQIRGLAALDDSHPPLRSEFSMPDCQKTDIGTLCPYDAVMKRLNDAVDRTAL
LPVHYTPHHHHHH
**SEQ ID NO: 11** 
DTPSSGWTLEKVVEVSRHGVRPPTESNVKTIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNRSISLHGPEVLAIIAETIQMSYSNNNPLSEVAFG
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 12** 
DTPSSGWTLEKVVEVSRHGVRPPTESNVKTIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNRSISLHGPEVLAIIAETIQMSYSNNNPLSEVAFG
HAKTAAEVAALMPLLTANYDFTNDLPYVAHRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 13** 
DTPSSGWTLEKVVEVSRHGVRPPTESNVKTIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSISLHGPEVLAIIAETIRMSYSNNNPLSEVAFG
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 14** 
DTPSSGWTLEKVVEVSRHGVRPPTESNVKTIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSISLHGPEVLAIIAETIQMSYSNNNPLSEVAFG
HAKTTAEVAALMPLLTANYDFTKDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
TLLPVHYTPHHHHHH
**SEQ ID NO: 15** 
DTPSSGWTLEKVVEVSRHGVRPPTESNVKTIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSISLHGPEVLAIIAETIRMSYSNNNPLSEVAFG
HAKTAAEVAALMPLLTANYDFTNDLPYVARRSASVLMNQIALALNTEHQAGAPPDAK
WLLYVAHDTNIAKLRTMLGFTWQMGDYPRGNIPPAGSLIFERWRNQQSGQRVVRIYF
QAQSLDQIRGLAALDDSHPPLRSEFSMPDCQKTDIGTLCPYDAVMKRLNDAVDRTAL
LPVHYTPHHHHHH
**SEQ ID NO: 16** 
DTPSSGWTLEKVVEVSRHGVRPPTESNVKTIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGHISLHGPEVLAIIAETIQMSYSNNNPLSEVAFG
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 17** 
DTPSSGWTLEKVVEVSRHGVRPPTESNVKTIQEGTDRQWPTWLTRYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSISLHGPEVLAIIAETIQMSYSNNNPLSEVAFG
HAKTAAEVAALMPLLTANYDFTNDLPYVARRGASVLMNQIALALNTEHQPDAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 18** 
DTPSSGWTLEKVVEVSRHGVRPPTESNVKTIQEGTDRQWPTWLTNYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSISLHGPEVLAIIAETIQMSYSNNNPLSEVAFG
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 19** 
DTPSSGWTLEKVVEVSRHGVRPPTESNVKTIQEGTDRQWPTWLTQYGELTGHGYAA
AELKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSIRLHGPEVLAIIAETIQMSYSNNNPLSEVAFG
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 20** 
DTPSSGWTLEKVVEVSRHGVRPPTESNVKTIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSISLHGPEVLAIIAETIQMSYSNNNPLSEVAFG
HAKTAAEVAALMPLLTANYDFTKDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 21** 
DTPSSGWTLEKVVEVSRHGVRPPTESNVKSIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSISLHGPEVLAIIAETIQMSYSNNNPLSEVAFG
HAKTAAEVAALMPLLTANYDFTKDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 22** 
DTPSSGWTLEKVVEVSRHGVRPPTESNVKTIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSISLHGPEVLAIIAETIRMSYSNNNPLSEVAFG
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLASLDDSHPPLRSEFSMPDCQKTDIGTLCPYDAVMKRLNDAVDRT
ALLPVHYTPHHHHHH
**SEQ ID NO: 23** 
DTPSSGWTLEKVVEVSRHGVRPPTEKNVKTIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSISLHGPEVLAIIAETIRESYSNNNPLSEVAFG
 
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 24** 
DTPSSGWTLEKVVEVSRHGVRPPTESNVKTIQEGTDRQWPTWLTQYGELTGHGYAA
AVLKGHYEGQYLRQHGLLCQACPAPGDVFVWASPLQRTRETAMALMDGVFPGCGV
AIQGPEDEKNDPLFHAETAGVTLDQQQVKADLQNAMQNKSAAQIQAQWQPAIDRLK
QAVCLPDKPCPAFSTPWEVKEDNNGRIYLHGPEVLANIAETIQMSYSNNNPLSEVAF
GHAKTAAEVAALMPLLTAKYDFTNDLPYVARRGASVLMNQIALALNTEHQADAPPD
AKWLLYVAHDTNIAYLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 25** 
DTPSSGWTLEKVVEVSRHGVRPPTESNVKAIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGRIYLHGPEVLANIAETIQMSYSNNNPLSEVAF
GHAKTAAEVAALMPLLTAKYDFTNDLPYVARRGASVLMNQIALALNTEHQADAPPD
AKWLLYVAHDTNIAYLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 26** 
DTPSSGWTLEKVVEVSRHGVRPPTESNVKEIQEGTDRQWPTWLTQYGELTGHGYAA
AVLKGHYEGQYLRQHGLLCQACPAPGDVFVWASPLQRTRETAMALMDGVFPGCGV
AIQGPEDEKNDPLFHAETAGVTLDQQQVKADLQNAMQNKSAAQIQAQWQPAIDRLK
QAVCLPDKPCPAFSTPWEVKEDNNGKIYLHGPEVLASIAETIQMSYSNNNPLSEVAF
GHAKTAAEVAALMPLLTAKYDFTNDLPYVARRGASVLMNQIALALNTEHQADAPPD
AKWLLYVAHDTNIAYLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 27** 
DTPSSGWTLEKVVEVSRHGVRPPTESNVKTIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAEKAGVTLDQQQVKADLQNAMQNKSAAQIQAQWQPAIDRLK
QAVCLPDKPCPAFSTPWEVKEDNNGSISLHGPEVLAIIAETIQMSYSNNNPLSEVAFG
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 28** 
DTPSSGWTLEKVVEVSRHGVRPPTESNVKTIQEGTNRQWPTWLTQYGELTGHGYAA
AVLKGHYEGQYLRQHGLLCQACPAPGDVFVWASPLQRTRETAMALMDGVFPGCGV
AIQGPEDEKNDPLFHAETAGVTLDQQQVKADLQNAMQNKSAAQIQAQWQPAIDRLK
QAVCLPDKPCPAFSTPWEVKEDNNGRIYLHGPEVLANIAETIQMSYSNNNPLSEVAF
GHAKTAAEVAALMPLLTAKYDFTNDLPYVARRGASVLMNQIALALNTEHQADAPPD
AKWLLYVAHDTNIAYLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 29** 
DTPSSGWTLEKVVEVSRHGVRPPTESNVKAIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAEKAGVTLDQQQVKADLQNAMQNKSAAQIQAQWQPAIDRLK
QAVCLPDKPCPAFSTPWEVKEDNNGRIYLHGPEVLANIAETIQMSYSNNNPLSEVAF
GHAKTAAEVAALMPLLTAKYDFTNDLPYVARRGASVLMNQIALALNTEHQADAPPD
AKWLLYVAHDTNIAYLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 30** 
DTPSSGWTLEKVVEVSRHGVRPPTESNVKTIQEGTNRQWPTWLTQYGELTGHGYAA
AVLKGHYEGQYLRQHGLLCQACPAPGDVFVWASPLQRTRETAMALMDGVFPGCGV
AIQGPEDEDNDPLFHAEKAGVTLDQQQVKADLQNAMQNKSAAQIQAQWQPAIDRLK
QAVCLPDKPCPAFSTPWEVKEDNNGRIYLHGPEVLANIAETIQMSYSNNNPLSEVAF
GHAKTAAEVAALMPLLTAKYDFTNDLPYVARRGASVLMNQIALALNTEHQADAPPD
AKWLLYVAHDTNIAYLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 31** 
DTPSSGWTLEKVVEVSRHGVRPPTESNVKTIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAEKAGVTLDQQQVKADLQNAMQNKSAAQIQAQWQPAIDRLK
QAVCLPDKPCPAFSTPWEVKEDNNGRIYLHGPEVLANIAETIQMSYSNNNPLSEVAF
GHAKTAAEVAALMPLLTAKYDFTNDLPYVARRGASVLMNQIALALNTEHQADAPPD
AKWLLYVAHDTNIAYLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 32** 
DTPSSGWTLEKVVEVSRHGVRPPTENNVKTIQEGTNRQWPTWLTQYGELTGHGYAA
AVLKGHYEGQYLRQHGLLCQACPAPGDVFVWASPLQRTRETAMALMDGVFPGCGV
AIQGPEDEDNDPLFHAEKAGVTLDQQQVKADLQNAMQNKSAAQIQAQWQPAIDRLK
 
QAVCLPDKPCPAFSTPWEVKEDNNGSISLHGPEVLAIIAETIQMSYSNNNPLSEVAFG
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 33** 
DTPSSGWTLEKVVEVSRHGVRPPTASNVKKIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGHISLHGPEVLAIIAETIQMSYSNNNPLSEVAFG
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 34** 
DTPSSGWTLEKVVEVSRHGVRPPTESNVKTIQEGTDRQWPTWLTQYGELTGHGYAA
AELKGHYEGQYLRQHGLLCQACPAPGDVFVWASPLQRTRETAMALMDGVFPGCGV
AIQGPEDEDNDPLFHAEKAGVTLDQQQVKADLQNAMQNKSAAQIQAQWQPAIDRLK
QAVCLPDKPCPAFSTPWEVKEDNNGRIYLHGPEVLANIAETIQMSYSNNNPLSEVAF
GHAKTAAEVAALMPLLTAKYDFTNDLPYVARRGASVLMNQIALALNTEHQADAPPD
AKWLLYVAHDTNIAYLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 35** 
DTPSSGWTLEKVVEVSRHGVRPPTENNVKTIQEGTNRQWPTWLTQYGELTGHGYAA
AVLKGHYEGQYLRQHGLLCQACPAPGDVFVWASPLQRTRETAMALMDGVFSGCGVA
IQGPEDEDNDPLFHAEKAGVTLDQQQVKADLQNAMQNKSAAQIQAQWQPAIDRLKQ
AVCLPDKPCPAFSTPWEVKEDNNGSISLHGPEVLAIIAETIQMSYSNNNPLSEVAFGH
AKTAAEVAALMPLLTANYDFTNDLPYVARRGASVLMNQIALALNTEHQADAPPDAK
WLLYVAHDTNIAKLRTMLGFTWQMGDYPRGNIPPAGSLIFERWRNQQSGQRVVRIYF
QAQSLDQIRGLAALDDSHPPLRSEFSMPDCQKTDIGTLCPYDAVMKRLNDAVDRTAL
LPVHYTPHHHHHH
**SEQ ID NO: 36** 
DTPSSGWTLEKVVEVSRHGVRPPTESNVKTIQEGTDRQWPTWLTQYGELTGHGYAA
AELKGHYEGQYLRQHGLLCQACPAPGDVFVWASPLQRTRETAMALMDGVFPGCGV
AIQGPEDEDNDPLFHAEKAGVTLDQQQVKADLQNAMQNKSAAQIQAQWQPAIDRLK
QAVCLPDKPCPAFSTPWEVKEDNNGKIYLHGPEVLAIIAETIQMSYSNNNPLSEVAFG
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
 
YFQAQSLDQIRGLAALDDSHPPLRSEFSMPDCQKTDIGTLCPYDAVMKRLNDAVDRT
ALLPVHYTPHHHHHH
**SEQ ID NO: 37** 
DTPSSGWTLEKVVEVSRHGVRPPTESNVKTIQEGTDRQWPTWLTQYGELTGHGYAA
AELKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSIRLHGPEVLAIIAETIQMSYSNNNPLSEVAFG
HAKTAAEVAALLPLLTANYDFTNDLPYVARRGASVLMNQIALALNTEHQADAPPDAK
WLLYVAHDTNIAKLRTMLGFTWQMGDYPRGNIPPAGSLIFERWRNQQSGQRVVRIYF
QAQSLDQIRGLAALDDSHPPLRSEFSMPDCQKTDIGTLCPYDAVMKRLNDAVDRTAL
LPVHYTPHHHHHH
**SEQ ID NO: 38** 
DTPSSGWTLEKVVEVSRHGVRPPTESNVKTIQEGTDRQWPTWLTQYGELTGHGYAA
AELKGHYEGQYLRQHGLLCQACPAPGDVFVWASPLQRTRETAMALMDGVFPGCGV
GIQGPEDEDNDPLFHAETAGVTLDQQQVKADLQNAMQNKSAAQIQAQWQPAIDRLK
QAVCLPDKPCPAFSTPWEVKEDNNGSIRLHGPEVLAIIAETIQMSYSNNNPLSEVAFG
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 39** 
DTPSSGWTLEKVVEVSRHGVRPPTASNVAKIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSITLHGPEVLAIIAETIQMSYSNNNPLSEVAFG
HAKTAAEVAALMPLLTANYDFTKDLPYVARRGASVLMNQIALALNTEHQADAPPDA
KWLLYVAHDTNIAMLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVRYTPHHHHHH
**SEQ ID NO: 40** 
DTPSSGWTLEKVVEVSRHGVRPPTASNVAKIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVEEDNNGSISLHGPEVLAIIAETIQMSYSNNNPLSEVAFG
HAKTAAEVAALMPLLTANYDFTKDLPYVARRGASVLMNQIALALNTEHQADAPPDA
KWLLYVAHDTNIAMLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 41** 
ETTSSGWTLEKVVEVSRHGVRPPTASNVAKIQEGTDRQWPTWLTQYGELTGHGYAA
AVLKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSITLHGPEVLANIAETIQMSYSNDNPLSEVAF
GHAKTAAEVAALMPLLTAKYDFTNDLPYVARRGASVLMNQIALALNTEHQADAPPD
AKWLLYVAHDTNIAMLRTMLGFTWQMGDYPRGNIPPAGSLIFERWRNQQSGQRVVR
IYFQAQSLDQIRGLAALDDSHPPLRSEFSMPDCQKTDIGTLCPYDAVMKRLNDAVDR
TALLPVRYTPHHHHHH
**SEQ ID NO: 42** 
DTPSSGWTLEKVVEVSRHGVRPPTESNVKTIQEGTDRQWPTWLTQYGELTGHGYAA
AELKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSIRLHGPEVLAIIAETIQMSYANNNPLSEVAFG
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 43** 
DTPSSGWTLEKVVEVSRHGVRPPTEGNVKAIQEGTDRQWPTWLTQYGELTGHGYAA
AELKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSIRLHGPEVLAIIAETIQMSYSNNNPLSEVAFG
HAKTAAEVAALMPLLTANYDFTKDLPYVARRGASVLMNQIALALNTEHQADAPPDA
KWLLYVAHDTNIAKLRTMLGFTWQMGDYPRGNIPPAGSLIFERWRNQQSGDRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 44** 
DTPSSGWTLEKVVEVSRHGVRPPTESNVKTIQEGTDRQWPTWLTQYGELTGHGYAA
AELKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSIRLHGPEVLAIIAETIRMSYSNNNPLSEVAFG
HAKTAAEVAALMPLLTANYDFTNDLPYVARRGASVLMNQIALALNTEHQADAPPDA
KWLLYVAHDTNIAKLRTMLGFTWQMGDYPRGNIPPAGSLIFERWRNQQSGQRVVRI
YFQAQSLDQIRGLAALDDSHPPLRSEFSMPDCQKTDIGTLCPYDAVMKRLNDAVDRT
ALLPVHYTPHHHHHH
**SEQ ID NO: 45** 
DTPSAAAPSSGWTLEKVVEVSRHGVRPPTESNVKTIQEGTDRQWPTWLTQYGELTG
HGYAAAELKGHYEGQYLRQHGLLCQACPAPGDVFVWASPLQRTRETAMALMDGVF
PGCGVAIQGPEDEDNDPLFHAETAGVTLDQQQVKADLQNAMQNKSAAQIQAQWQPA
IDRLKQAVCLPDKPCPAFSTPWEVKEDNNGSIRLHGPEVLAIIAETIQMSYSNNNPLS
 
EVAFGHAKTAAEVAALMPLLTANYDFTNDLPYVARRGASVLMNQIALALNTEHQAD
APPDAKWLLYVAHDTNIAKLRTMLGFTWQMGDYPRGNIPPAGSLIFERWRNQQSGQ
RVVRIYFQAQSLDQIRGLAALDDSHPPLRSEFSMPDCQKTDIGTLCPYDAVMKRLND
AVDRTALLPVHYTPHHHHHH
**SEQ ID NO: 46** 
DTPSSGWTLEKVVEVSRHGVRPPTESNVKTIQEGTDRQWPTWLTQYGELTGHGYAA
AELKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSIRLHGPEVLAIIAETIQMSYSNNNPLSEVAFG
HAKTAAEVAALMPLLTANYDFTNDLPYVARRGASVLMNQIALALNTEHQAAAAQAD
APPDAKWLLYVAHDTNIAKLRTMLGFTWQMGDYPRGNIPPAGSLIFERWRNQQSGQ
RVVRIYFQAQSLDQIRGLAALDDSHPPLRSEFSMPDCQKTDIGTLCPYDAVMKRLND
AVDRTALLPVHYTPHHHHHH
**SEQ ID NO: 47**

DTPSSGWTLEKVVEVSRHGVRPPTESNVKTIQEGTDRQWPTWLTQYGELTGHGYAA
AELKGHYEGQYLRQHGLLCQACPAPGDVFVWASPLQRTRETAMALMDGVFPGCGV
AIQGPEDEDNDPLFHAETAGVTLDQQQVKADLQNAMQNKSAAQIQAQWQPAIDRLK
QAVCLPDKPCPAFSTPWEVKEDNNGSIRLHGPEVAAAEVLAIIAETIQMSYSNNNPLS
EVAFGHAKTAAEVAALMPLLTANYDFTNDLPYVARRGASVLMNQIALALNTEHQAD
APPDAKWLLYVAHDTNIAKLRTMLGFTWQMGDYPRGNIPPAGSLIFERWRNQQSGQ
RVVRIYFQAQSLDQIRGLAALDDSHPPLRSEFSMPDCQKTDIGTLCPYDAVMKRLND
AVDRTALLPVHYTPHHHHHH

### Examples

In the following the present invention is described by the examples. The examples are considered for illustrative purpose only and do not limit the scope of the present invention and claims in any respect.

### Example 1: Thermostability of 3-phytase mutants

Characterization of 3-phytase mutants regarding their thermostability was done by measuring the volumetric activity at pH 5.5 in acetate buffer before and after heat treatment. Heat treatment was done for 15 minutes at different temperatures (58 to 85 °C) and the enzymatic activity was determined by measuring the phosphate release at different time points using the photometric assay according to Example 4. Using the activities before and after heat treatment IC₅₀ values were determined, wherein IC₅₀ is defined as the temperature at which the enzyme shows 50% residual activity after 25 min. of incubation. All 3-phytase mutants of SEQ ID NO: 2 show significantly higher thermostability than the wildtype SEQ ID NO: 2 as presented in the following:

| **SEQ ID NO** | **IC₅₀ [°C]** |
|---|---|
| SEQ ID. NO: 2 | 58 |
| SEQ ID. NO: 1 | 81 |
| SEQ ID. NO: 3 | 80 |
| SEQ ID. NO: 4 | 80 |
| SEQ ID. NO: 5 | 79 |
| SEQ ID. NO: 6 | 79 |
| SEQ ID. NO: 7 | 79 |
| SEQ ID. NO: 8 | 79 |
| SEQ ID. NO: 9 | 78 |
| SEQ ID. NO: 10 | 77 |
| SEQ ID. NO: 11 | 77 |
| SEQ ID. NO: 12 | 74 |
| SEQ ID. NO: 13 | 73 |
| SEQ ID. NO: 14 | 73 |
| SEQ ID. NO: 15 | 71 |
| SEQ ID. NO: 16 | 79 |
| SEQ ID. NO: 17 | 79 |
| SEQ ID. NO: 18 | 79 |
| SEQ ID. NO: 19 | 78 |
| SEQ ID. NO: 20 | 76 |
| SEQ ID. NO: 21 | 75 |
| SEQ ID. NO: 22 | 74 |
| SEQ ID. NO: 23 | 73 |
| SEQ ID. NO: 24 | 79 |
| SEQ ID. NO: 25 | 79 |
| SEQ ID. NO: 26 | 80 |
| SEQ ID. NO: 27 | 78 |
| SEQ ID. NO: 28 | 81 |
| SEQ ID. NO: 29 | 80 |
| SEQ ID. NO: 30 | 81 |
| SEQ ID. NO: 31 | 79 |
| SEQ ID. NO: 32 | 80 |
| SEQ ID. NO: 33 | 79 |
| SEQ ID. NO: 34 | 79 |
| SEQ ID. NO: 35 | 79 |
| SEQ ID. NO: 36 | 79 |
| SEQ ID. NO: 37 | 79 |
| SEQ ID. NO: 38 | 78 |
| SEQ ID. NO: 39 | 76 |
| SEQ ID. NO: 40 | 75 |
| SEQ ID. NO: 41 | 73 |
| SEQ ID. NO: 42 | 76 |
| SEQ ID. NO: 43 | 75 |
| SEQ ID. NO: 44 | 73 |
| SEQ ID. NO: 45 | 76 |
| SEQ ID. NO: 46 | 75 |
| SEQ ID. NO: 47 | 64 |

### Example 2: Phosphate release in application testing of thermostable 3-phytase mutants comprising the mutations D149N, S189D, K190N, K191N, R210Q and L211M

The phosphate release of each 3-phytase mutant of SEQ ID NO: 2 comprising the mutations D149N, S189D, K190N, K191N, R210Q and L211M was determined in a close-to-application testing and compared to the wildtype SEQ ID NO:2. Therefore, 3-phytase mutants were expressed in *Pichia pastoris* and the volumetric activity was determined according to DIN EN ISO 30024:2009 (https://www.iso.org/standard/45787.html) with minor changes (format downscaled by a factor of 5). The enzymes were applied with 500 units/kg feed in the application testing. Here, 15 g of a 70/30 mixture of corn and soy was incubated at 41°C using 25% dry matter for 60 minutes at pH 3 and 5. Afterwards, the released phosphate was measured using the ammonium molybdate assay according to Example 4. The Performance Index was calculated as follows: [(Released phosphate [mM] at pH 3)ᵥₐᵣᵢₐₙₜ/ released phosphate [mM] at pH 3)_{SEQ ID NO: 2}] x [(released phosphate [mM] at pH 5)ᵥₐᵣᵢₐₙₜ / (released phosphate [mM] at pH 5) _{SEQ ID NO: 2}]. All 3-phytase mutants show an improved performance index compared to the wildtype SEQ ID NO: 2 as presented in the following Table:

| **SEQ ID NO** | **Performance Index** |
|---|---|
| SEQ ID. NO: 2 | 1,00 |
| SEQ ID. NO: 47 | 2,92 |
| SEQ ID. NO: 4 | 1,88 |
| SEQ ID. NO: 6 | 1,88 |
| SEQ ID. NO: 7 | 1,89 |
| SEQ ID. NO: 8 | 1,96 |
| SEQ ID. NO: 10 | 2,18 |
| SEQ ID. NO: 14 | 2,09 |
| SEQ ID. NO: 19 | 2,02 |
| SEQ ID. NO: 20 | 1,93 |
| SEQ ID. NO: 21 | 1,94 |
| SEQ ID. NO: 26 | 2,43 |
| SEQ ID. NO: 28 | 2,39 |
| SEQ ID. NO: 29 | 2,08 |
| SEQ ID. NO: 30 | 1,90 |
| SEQ ID. NO: 31 | 1,95 |
| SEQ ID. NO: 34 | 1,94 |
| SEQ ID. NO: 1 | 2,00 |
| SEQ ID. NO: 37 | 2,49 |
| SEQ ID. NO: 38 | 1,97 |
| SEQ ID. NO: 39 | 2,11 |
| SEQ ID. NO: 40 | 2,27 |
| SEQ ID. NO: 41 | 1,89 |
| SEQ ID. NO: 45 | 1,94 |
| SEQ ID. NO: 46 | 1,88 |

### Example 3: Determination of the pH profile of thermostable 3-phytase mutants of SEQ ID NO: 2

The improved performance of the 3-phytase mutants is majorly founded by a shifted pH profile towards a lower pH. Therefore, the pH profile of selected 3-phytase mutants of SEQ ID NO: 2 was determined by measuring the activity at different pH values using the photometric assay according to Example 4. Fig. shows a shift in pH optimum for all variants compared to the wildtype SEQ ID NO: 2.

### Example 4: Determination of the enzymatic activity of 3-phytase mutants

5 µL enzyme solution (in 100 mmol/L sodium acetate buffer, pH 5.5, 0.05 % (w/v) Triton X-100) were incubated with 95 µL 2,88 mmol/L sodium phytate (Sigma 68388, lot BCBM4006V) in 100 mmol/L sodium acetate buffer, pH 5.5 at for 25 min. The reaction was stopped by adding 100 µL 10 % (w/v) trichloroacetic acid. Subsequently, 100 µL of the stopped enzymatic reaction was mixed with 100 µL molybdate reagent (aqueous solution of 1.2 % (w/v) ammonium molybdate; 4.4 % (v/v) sulfuric acid, and 27 mg/mL (w/v) ferrous sulfate) and the solution was incubated for 15 min at room temperature. Absorbance at 700 nm was determined and the amount of released inorganic phosphate was calculated using inorganic phosphate standard solutions (0 - 1.5 mmol/L). One unit of phytase activity was defined as the amount of enzyme that releases 1 µmol phosphate per min at 37 °C.

## Claims

1. Bio-efficacious phytase mutant having sequence identity of at least 96,25 % around SEQ ID NO. 2, wherein the mutant comprises a sequence of SEQ ID NO. 2 and a mutation selected from the group consisting of D149N, S189D, K190N, K191N, R210Q, L211E, L211M, K25E, K25A, G26N, G26S, N193H, N193K, N193S, N193R, D1E, P3T, G26K, K29A, T30S, T30A, T30K, D36N, Q45N, Q45R, V58E, S75C, Q76R, P107S, A112G, D120K, T129K, Q136R, Q144L, D149N, K187E, D189N, G192R, S195R, S195Y, S195T, S195N, N204I, N204T, N204S, S214A, N216D, A230T, M237L, N243K, N248K, R255H, G257S, F264Y, A275P, D276G, K295Y, K295M, Q332D, A352S, V383M, R393H, A395T and combinations thereof.

2. Bio-efficacious phytase mutant according to claim 1 comprising mutations D149N, S189D, K190N and K191N.

3. Bio-efficacious phytase mutant according to claim 1 or 2 comprising mutations R210Q and L211M.

4. Bio-efficacious phytase mutant according to any one of claims 1 to 3 comprising mutations K25E or K25A, and G26N or G26S, and N193S or N193R.

5. Bio-efficacious phytase mutant according to any one of claims 1 to 4 comprising mutations at positions 25, 26, 193, 210 and/or 211.

6. Bio-efficacious phytase mutant according to any one of claims 1 to 5, which is active up to 90 °C.

7. Bio-efficacious phytase mutant according to any one of claims 1 to 6, which is active at a pH from 3 to 5.5.

8. Use of the bio-efficacious phytase mutant according to any one of claims 1 to 7 for the production of a food or feed product.


SEQUENCE LISTING

<110> Clariant Produkte (Deutschland) GmbH

<120> Optimized 3-phytase and its use in food or feed production

<130> 2018DE612-EP-EPA

<160> 47

<170> BiSSAP 1.3.6

<210> 1
<211> 409
<212> PRT
<213> Artificial Sequence

<220>
<223> Variant of wt Sequence (Seq2)

<400> 1
   Asp Thr Pro Ser Ser Gly Trp Thr Leu Glu Lys Val Val Glu Val Ser
   1               5                   10                  15
   Arg His Gly Val Arg Pro Pro Thr Glu Asn Asn Val Lys Thr Ile Gln
               20                  25                  30
   Glu Gly Thr Asn Arg Gln Trp Pro Thr Trp Leu Thr Gln Tyr Gly Glu
           35                  40                  45
   Leu Thr Gly His Gly Tyr Ala Ala Ala Val Leu Lys Gly His Tyr Glu
       50                  55                  60
   Gly Gln Tyr Leu Arg Gln His Gly Leu Leu Cys Gln Ala Cys Pro Ala
   65                  70                  75                  80
   Pro Gly Asp Val Phe Val Trp Ala Ser Pro Leu Gln Arg Thr Arg Glu
                   85                  90                  95
   Thr Ala Met Ala Leu Met Asp Gly Val Phe Ser Gly Cys Gly Val Ala
               100                 105                 110
   Ile Gln Gly Pro Glu Asp Glu Asp Asn Asp Pro Leu Phe His Ala Glu
           115                 120                 125
   Lys Ala Gly Val Thr Leu Asp Gln Gln Gln Val Lys Ala Asp Leu Gln
       130                 135                 140
   Asn Ala Met Gln Asn Lys Ser Ala Ala Gln Ile Gln Ala Gln Trp Gln
   145                 150                 155                 160
   Pro Ala Ile Asp Arg Leu Lys Gln Ala Val Cys Leu Pro Asp Lys Pro
                   165                 170                 175
   Cys Pro Ala Phe Ser Thr Pro Trp Glu Val Lys Glu Asp Asn Asn Gly
               180                 185                 190
   Arg Ile Tyr Leu His Gly Pro Glu Val Leu Ala Asn Ile Ala Glu Thr
           195                 200                 205
   Ile Gln Met Ser Tyr Ser Asn Asn Asn Pro Leu Ser Glu Val Ala Phe
       210                 215                 220
   Gly His Ala Lys Thr Ala Ala Glu Val Ala Ala Leu Met Pro Leu Leu
   225                 230                 235                 240
   Thr Ala Lys Tyr Asp Phe Thr Asn Asp Leu Pro Tyr Val Ala Arg Arg
                   245                 250                 255
   Gly Ala Ser Val Leu Met Asn Gln Ile Ala Leu Ala Leu Asn Thr Glu
               260                 265                 270
   His Gln Ala Asp Ala Pro Pro Asp Ala Lys Trp Leu Leu Tyr Val Ala
           275                 280                 285
   His Asp Thr Asn Ile Ala Tyr Leu Arg Thr Met Leu Gly Phe Thr Trp
       290                 295                 300
   Gln Met Gly Asp Tyr Pro Arg Gly Asn Ile Pro Pro Ala Gly Ser Leu
   305                 310                 315                 320
   Ile Phe Glu Arg Trp Arg Asn Gln Gln Ser Gly Gln Arg Val Val Arg
   325 330 335
   Ile Tyr Phe Gln Ala Gln Ser Leu Asp Gln Ile Arg Gly Leu Ala Ala
   340 345 350
   Leu Asp Asp Ser His Pro Pro Leu Arg Ser Glu Phe Ser Met Pro Asp
   355 360 365
   Cys Gln Lys Thr Asp Ile Gly Thr Leu Cys Pro Tyr Asp Ala Val Met
   370 375 380
   Lys Arg Leu Asn Asp Ala Val Asp Arg Thr Ala Leu Leu Pro Val His
   385 390 395 400
   Tyr Thr Pro His His His His His His
   405

<210> 2
<211> 409
<212> PRT
<213> Artificial Sequence

<220>
<223> wt sequence of Dickeya zeae phytase with His tag

<400> 2
Asp Thr Pro Ser Ser Gly Trp Thr Leu Glu Lys Val Val Glu Val Ser
1               5                   10                  15
Arg His Gly Val Arg Pro Pro Thr Lys Gly Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Ser Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asp Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Ser Lys Lys Gly
            180                 185                 190
Asn Ile Ser Leu His Gly Pro Glu Val Leu Ala Asn Ile Ala Glu Thr
        195                 200                 205
Ile Arg Leu Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

                    325                     330                     335
Ile Tyr Phe Gln Ala Gln Ser Leu Asp Gln Ile Arg Gly Leu Ala Ala
            340                     345                     350
Leu Asp Asp Ser His Pro Pro Leu Arg Ser Glu Phe Ser Met Pro Asp
        355                     360                     365
Cys Gln Lys Thr Asp Ile Gly Thr Leu Cys Pro Tyr Asp Ala Val Met
    370                     375                     380
Lys Arg Leu Asn Asp Ala Val Asp Arg Thr Ala Leu Leu Pro Val His
385                     390                     395                     400
Tyr Thr Pro His His His His His His
                405

<210> 3
<211> 409
<212> PRT
<213> Artificial Sequence

<220>
<223> Variant of wt sequence (Seq2)

<400> 3
Asp Thr Pro Ser Ser Gly Trp Thr Leu Glu Lys Val Val Glu Val Ser
1               5                   10                  15
Arg His Gly Val Arg Pro Pro Thr Glu Asn Asn Val Lys Thr Ile Gln
            20                  25                  30
Glu Gly Thr Asn Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Met Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 4
<211> 409
<212> PRT
<213> Artificial Sequence

<220>
<223> Variant of wt sequence (Seq2)

<400> 4
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asn Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

                325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 5
<211> 409
<212> PRT
<213> Artificial Sequence

<220>
<223> Variant of wt sequence (Seq2)

<400> 5
Asp Thr Pro Ser Ser Gly Trp Thr Leu Glu Lys Val Val Glu Val Ser
1               5                   10                  15
Arg His Gly Val Arg Pro Pro Thr Glu Asn Asn Val Lys Thr Ile Gln
            20                  25                  30
Glu Gly Thr Asn Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Ser Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

                    325                     330                     335
Ile Tyr Phe Gln Ala Gln Ser Leu Asp Gln Ile Arg Gly Leu Ala Ala
            340                     345                     350
Leu Asp Asp Ser His Pro Pro Leu Arg Ser Glu Phe Ser Met Pro Asp
        355                     360                     365
Cys Gln Lys Thr Asp Ile Gly Thr Leu Cys Pro Tyr Asp Ala Val Met
    370                     375                     380
Lys Arg Leu Asn Asp Ala Val Asp Arg Thr Ala Leu Leu Pro Val His
385                     390                     395                     400
Tyr Thr Pro His His His His His His
                405

<210> 6
<211> 409
<212> PRT
<213> Artificial Sequence

<220>
<223> Variant of wt sequence (Seq2)

<400> 6
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Arg Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 7
<211> 409
<212> PRT
<213> Artificial Sequence

<220>
<223> Variant of wt sequence (Seq2)

<400> 7
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Leu
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 8
<211> 409
<212> PRT
<213> Artificial Sequence

<220>
<223> Variant of wt sequence (Seq2)

<400> 8
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asn Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp His Thr Ala Leu Leu Pro Val His
385                 390                 395                 400
Tyr Thr Pro His His His His His His
                405

<210> 9
<211> 409
<212> PRT
<213> Artificial Sequence

<220>
<223> Variant of wt sequence (Seq2)

<400> 9
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Ser Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Lys Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 10
<211> 409
<212> PRT
<213> Artificial Sequence

<220>
<223> Variant of wt sequence (Seq2)

<400> 10
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Arg Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Arg Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Thr Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Tyr Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 11
<211> 409
<212> PRT
<213> Artificial Sequence

<220>
<223> Variant of wt sequence (Seq2)

<400> 11
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Arg
            180                 185                 190
Ser Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 12
<211> 409
<212> PRT
<213> Artificial Sequence

<220>
<223> Variant of wt sequence (Seq2)

<400> 12
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Arg
            180                 185                 190
Ser Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala His Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

                    325                     330                     335
Ile Tyr Phe Gln Ala Gln Ser Leu Asp Gln Ile Arg Gly Leu Ala Ala
            340                     345                     350
Leu Asp Asp Ser His Pro Pro Leu Arg Ser Glu Phe Ser Met Pro Asp
        355                     360                     365
Cys Gln Lys Thr Asp Ile Gly Thr Leu Cys Pro Tyr Asp Ala Val Met
    370                     375                     380
Lys Arg Leu Asn Asp Ala Val Asp Arg Thr Ala Leu Leu Pro Val His
385                     390                     395                     400
Tyr Thr Pro His His His His His His
                405

<210> 13
<211> 409
<212> PRT
<213> Artificial Sequence

<220>
<223> Variant of wt sequence (Seq2)

<400> 13
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Arg Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 14
<211> 409
<212> PRT
<213> Artificial Sequence

<220>
<223> Variant of wt sequence (Seq2)

<400> 14
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Thr Ala Glu Val Ala Ala Leu Met Pro Leu Leu
225                 230                 235                 240
Thr Ala Asn Tyr Asp Phe Thr Lys Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Thr Leu Leu Pro Val His
385                 390                 395                 400
Tyr Thr Pro His His His His His His
                405

<210> 15
<211> 409
<212> PRT
<213> Artificial Sequence

<220>
<223> Variant of wt sequence (Seq2)

<400> 15
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Arg Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Ser Ala Ser Val Leu Met Asn Gln Ile Ala Leu Ala Leu Asn Thr Glu
            260                 265                 270
His Gln Ala Gly Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 16
<211> 409
<212> PRT
<213> Artificial Sequence

<220>
<223> Variant of wt sequence (Seq2)

<400> 16
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
His Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 17
<211> 409
<212> PRT
<213> Artificial Sequence

<220>
<223> Variant of wt sequence (Seq2)

<400> 17
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Arg Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Pro Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 18
<211> 409
<212> PRT
<213> Artificial Sequence

<220>
<223> Variant of wt sequence (Seq2)

<400> 18
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Asn Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 19
<211> 409
<212> PRT
<213> Artificial Sequence

<220>
<223> Variant of wt sequence (Seq2)

<400> 19
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Glu Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Arg Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 20
<211> 409
<212> PRT
<213> Artificial Sequence

<220>
<223> Variant of wt sequence (Seq2)

<400> 20
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Lys Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 21
<211> 409
<212> PRT
<213> Artificial Sequence

<220>
<223> Variant of wt sequence (Seq2)

<400> 21
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Ser Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Lys Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 22
<211> 409
<212> PRT
<213> Artificial Sequence

<220>
<223> Variant of wt sequence (Seq2)

<400> 22
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Arg Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ser
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 23
<211> 409
<212> PRT
<213> Artificial Sequence

<220>
<223> Variant of wt sequence (Seq2)

<400> 23
Asp Thr Pro Ser Ser Gly Trp Thr Leu Glu Lys Val Val Glu Val Ser
1               5                   10                  15
Arg His Gly Val Arg Pro Pro Thr Glu Lys Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Arg Glu Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

                325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 24
<211> 409
<212> PRT
<213> Artificial Sequence

<220>
<223> Variant of wt sequence (Seq2)

<400> 24
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Lys Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Arg Ile Tyr Leu His Gly Pro Glu Val Leu Ala Asn Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Lys Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Tyr Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

                    325                     330                     335
Ile Tyr Phe Gln Ala Gln Ser Leu Asp Gln Ile Arg Gly Leu Ala Ala
            340                     345                     350
Leu Asp Asp Ser His Pro Pro Leu Arg Ser Glu Phe Ser Met Pro Asp
        355                     360                     365
Cys Gln Lys Thr Asp Ile Gly Thr Leu Cys Pro Tyr Asp Ala Val Met
    370                     375                     380
Lys Arg Leu Asn Asp Ala Val Asp Arg Thr Ala Leu Leu Pro Val His
385                     390                     395                     400
Tyr Thr Pro His His His His His His
                405

<210> 25
<211> 409
<212> PRT
<213> Artificial Sequence

<220>
<223> Variant of wt sequence (Seq2)

<400> 25
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Ala Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Arg Ile Tyr Leu His Gly Pro Glu Val Leu Ala Asn Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Lys Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Tyr Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 26
<211> 409
<212> PRT
<213> Artificial Sequence

<220>
<223> Variant of wt sequence (Seq2)

<400> 26
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Glu Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Lys Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Lys Ile Tyr Leu His Gly Pro Glu Val Leu Ala Ser Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Lys Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Tyr Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

                325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 27
<211> 409
<212> PRT
<213> Artificial Sequence

<220>
<223> Variant of wt sequence (Seq2)

<400> 27
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Lys Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 28
<211> 409
<212> PRT
<213> Artificial Sequence

<220>
<223> Variant of wt sequence (Seq2)

<400> 28
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asn Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Lys Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Arg Ile Tyr Leu His Gly Pro Glu Val Leu Ala Asn Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Lys Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Tyr Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 29
<211> 409
<212> PRT
<213> Artificial Sequence

<220>
<223> Variant of wt sequence (Seq2)

<400> 29
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Ala Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Lys Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Arg Ile Tyr Leu His Gly Pro Glu Val Leu Ala Asn Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Lys Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Tyr Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 30
<211> 409
<212> PRT
<213> Artificial Sequence

<220>
<223> Variant of wt sequence (Seq2)

<400> 30
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asn Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Lys Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Arg Ile Tyr Leu His Gly Pro Glu Val Leu Ala Asn Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Lys Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Tyr Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 31
<211> 409
<212> PRT
<213> Artificial Sequence

<220>
<223> Variant of wt sequence (Seq2)

<400> 31
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Lys Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Arg Ile Tyr Leu His Gly Pro Glu Val Leu Ala Asn Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Lys Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Tyr Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 32
<211> 409
<212> PRT
<213> Artificial Sequence

<220>
<223> Variant of wt sequence (Seq2)

<400> 32
Asp Thr Pro Ser Ser Gly Trp Thr Leu Glu Lys Val Val Glu Val Ser
1               5                   10                  15
Arg His Gly Val Arg Pro Pro Thr Glu Asn Asn Val Lys Thr Ile Gln
            20                  25                  30
Glu Gly Thr Asn Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Lys Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 33
<211> 409
<212> PRT
<213> Artificial Sequence

<220>
<223> Variant of wt sequence (Seq2)

<400> 33
Asp Thr Pro Ser Ser Gly Trp Thr Leu Glu Lys Val Val Glu Val Ser
1               5                   10                  15
Arg His Gly Val Arg Pro Pro Thr Ala Ser Asn Val Lys Lys Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
His Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 34
<211> 409
<212> PRT
<213> Artificial Sequence

<220>
<223> Variant of wt sequence (Seq2)

<400> 34
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Glu Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Lys Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Arg Ile Tyr Leu His Gly Pro Glu Val Leu Ala Asn Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Lys Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Tyr Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 35
<211> 409
<212> PRT
<213> Artificial Sequence

<220>
<223> Variant of wt sequence (Seq2)

<400> 35
Asp Thr Pro Ser Ser Gly Trp Thr Leu Glu Lys Val Val Glu Val Ser
1               5                   10                  15
Arg His Gly Val Arg Pro Pro Thr Glu Asn Asn Val Lys Thr Ile Gln
            20                  25                  30
Glu Gly Thr Asn Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Ser Gly Cys Gly Val Ala
            100                 105                 110
Ile Gln Gly Pro Glu Asp Glu Asp Asn Asp Pro Leu Phe His Ala Glu
        115                 120                 125
Lys Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 36
<211> 409
<212> PRT
<213> Artificial Sequence

<220>
<223> Variant of wt sequence (Seq2)

<400> 36
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Glu Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Lys Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Lys Ile Tyr Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 37
<211> 409
<212> PRT
<213> Artificial Sequence

<220>
<223> Variant of wt sequence (Seq2)

<400> 37
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Glu Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Arg Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Leu Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 38
<211> 409
<212> PRT
<213> Artificial Sequence

<220>
<223> Variant of wt sequence (Seq2)

<400> 38
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Glu Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Gly
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Arg Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 39
<211> 409
<212> PRT
<213> Artificial Sequence

<220>
<223> Variant of wt sequence (Seq2)

<400> 39
Asp Thr Pro Ser Ser Gly Trp Thr Leu Glu Lys Val Val Glu Val Ser
1               5                   10                  15
Arg His Gly Val Arg Pro Pro Thr Ala Ser Asn Val Ala Lys Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Thr Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Lys Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Met Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

                    325                     330                     335
Ile Tyr Phe Gln Ala Gln Ser Leu Asp Gln Ile Arg Gly Leu Ala Ala
            340                     345                     350
Leu Asp Asp Ser His Pro Pro Leu Arg Ser Glu Phe Ser Met Pro Asp
        355                     360                     365
Cys Gln Lys Thr Asp Ile Gly Thr Leu Cys Pro Tyr Asp Ala Val Met
    370                     375                     380
Lys Arg Leu Asn Asp Ala Val Asp Arg Thr Ala Leu Leu Pro Val Arg
385                     390                     395                     400
Tyr Thr Pro His His His His His His
                405

<210> 40
<211> 409
<212> PRT
<213> Artificial Sequence

<220>
<223> Variant of wt sequence (Seq2)

<400> 40
Asp Thr Pro Ser Ser Gly Trp Thr Leu Glu Lys Val Val Glu Val Ser
1               5                   10                  15
Arg His Gly Val Arg Pro Pro Thr Ala Ser Asn Val Ala Lys Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Glu Glu Asp Asn Asn Gly
            180                 185                 190
Ser Ile Ser Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Lys Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Met Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

                325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 41
<211> 409
<212> PRT
<213> Artificial Sequence

<220>
<223> Variant of wt sequence (Seq2)

<400> 41
Glu Thr Thr Ser Ser Gly Trp Thr Leu Glu Lys Val Val Glu Val Ser
1               5                   10                  15
Arg His Gly Val Arg Pro Pro Thr Ala Ser Asn Val Ala Lys Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Val Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Thr Leu His Gly Pro Glu Val Leu Ala Asn Ile Ala Glu Thr
        195                 200                 205
Ile Gln Met Ser Tyr Ser Asn Asp Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Lys Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Met Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val Arg
385                 390                 395                 400
Tyr Thr Pro His His His His His His
                405

<210> 42
<211> 409
<212> PRT
<213> Artificial Sequence

<220>
<223> Variant of wt sequence (Seq2)

<400> 42
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Glu Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Arg Leu His Gly Pro Glu Val Leu Ala Ile Ile Ala Glu Thr
        195                 200                 205
Ile Gln Met Ser Tyr Ala Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 43
<211> 409
<212> PRT
<213> Artificial Sequence

<220>
<223> Variant of wt sequence (Seq2)

<400> 43
Asp Thr Pro Ser Ser Gly Trp Thr Leu Glu Lys Val Val Glu Val Ser
1               5                   10                  15
Arg His Gly Val Arg Pro Pro Thr Glu Gly Asn Val Lys Ala Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Glu Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Arg Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Lys Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Asp Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 44
<211> 409
<212> PRT
<213> Artificial Sequence

<220>
<223> Variant of wt sequence (Seq2)

<400> 44
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Glu Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Arg Leu His Gly Pro Glu Val Leu Ala Ile Ile Ala Glu Thr
        195                 200                 205
Ile Arg Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Asp Ala Pro Pro Asp Ala Lys Trp Leu Leu Tyr Val Ala
        275                 280                 285
His Asp Thr Asn Ile Ala Lys Leu Arg Thr Met Leu Gly Phe Thr Trp
    290                 295                 300
Gln Met Gly Asp Tyr Pro Arg Gly Asn Ile Pro Pro Ala Gly Ser Leu
305                 310                 315                 320
Ile Phe Glu Arg Trp Arg Asn Gln Gln Ser Gly Gln Arg Val Val Arg

325                 330                 335
Ile Tyr Phe Gln Ala Gln Ser Leu Asp Gln Ile Arg Gly Leu Ala Ala
            340                 345                 350
Leu Asp Asp Ser His Pro Pro Leu Arg Ser Glu Phe Ser Met Pro Asp
        355                 360                 365
Cys Gln Lys Thr Asp Ile Gly Thr Leu Cys Pro Tyr Asp Ala Val Met
    370                 375                 380
Lys Arg Leu Asn Asp Ala Val Asp Arg Thr Ala Leu Leu Pro Val His
385                 390                 395                 400
Tyr Thr Pro His His His His His His
                405

<210> 45
<211> 414
<212> PRT
<213> Artificial Sequence

<220>
<223> Variant of wt sequence (Seq2)

<400> 45
Asp Thr Pro Ser Ala Ala Ala Pro Ser Ser Gly Trp Thr Leu Glu Lys
1               5                   10                  15
Val Val Glu Val Ser Arg His Gly Val Arg Pro Pro Thr Glu Ser Asn
            20                  25                  30
Val Lys Thr Ile Gln Glu Gly Thr Asp Arg Gln Trp Pro Thr Trp Leu
        35                  40                  45
Thr Gln Tyr Gly Glu Leu Thr Gly His Gly Tyr Ala Ala Ala Glu Leu
    50                  55                  60
Lys Gly His Tyr Glu Gly Gln Tyr Leu Arg Gln His Gly Leu Leu Cys
65                  70                  75                  80
Gln Ala Cys Pro Ala Pro Gly Asp Val Phe Val Trp Ala Ser Pro Leu
                85                  90                  95
Gln Arg Thr Arg Glu Thr Ala Met Ala Leu Met Asp Gly Val Phe Pro
            100                 105                 110
Gly Cys Gly Val Ala Ile Gln Gly Pro Glu Asp Glu Asp Asn Asp Pro
        115                 120                 125
Leu Phe His Ala Glu Thr Ala Gly Val Thr Leu Asp Gln Gln Gln Val
    130                 135                 140
Lys Ala Asp Leu Gln Asn Ala Met Gln Asn Lys Ser Ala Ala Gln Ile
145                 150                 155                 160
Gln Ala Gln Trp Gln Pro Ala Ile Asp Arg Leu Lys Gln Ala Val Cys
                165                 170                 175
Leu Pro Asp Lys Pro Cys Pro Ala Phe Ser Thr Pro Trp Glu Val Lys
            180                 185                 190
Glu Asp Asn Asn Gly Ser Ile Arg Leu His Gly Pro Glu Val Leu Ala
        195                 200                 205
Ile Ile Ala Glu Thr Ile Gln Met Ser Tyr Ser Asn Asn Asn Pro Leu
    210                 215                 220
Ser Glu Val Ala Phe Gly His Ala Lys Thr Ala Ala Glu Val Ala Ala
225                 230                 235                 240
Leu Met Pro Leu Leu Thr Ala Asn Tyr Asp Phe Thr Asn Asp Leu Pro
                245                 250                 255
Tyr Val Ala Arg Arg Gly Ala Ser Val Leu Met Asn Gln Ile Ala Leu
            260                 265                 270
Ala Leu Asn Thr Glu His Gln Ala Asp Ala Pro Pro Asp Ala Lys Trp
        275                 280                 285
Leu Leu Tyr Val Ala His Asp Thr Asn Ile Ala Lys Leu Arg Thr Met
    290                 295                 300
Leu Gly Phe Thr Trp Gln Met Gly Asp Tyr Pro Arg Gly Asn Ile Pro
305                 310                 315                 320
Pro Ala Gly Ser Leu Ile Phe Glu Arg Trp Arg Asn Gln Gln Ser Gly

325                 330                 335
Gln Arg Val Val Arg Ile Tyr Phe Gln Ala Gln Ser Leu Asp Gln Ile
            340                 345                 350
Arg Gly Leu Ala Ala Leu Asp Asp Ser His Pro Pro Leu Arg Ser Glu
        355                 360                 365
Phe Ser Met Pro Asp Cys Gln Lys Thr Asp Ile Gly Thr Leu Cys Pro
    370                 375                 380
Tyr Asp Ala Val Met Lys Arg Leu Asn Asp Ala Val Asp Arg Thr Ala
385                 390                 395                 400
Leu Leu Pro Val His Tyr Thr Pro His His His His His His
                405                 410

<210> 46
<211> 414
<212> PRT
<213> Artificial Sequence

<220>
<223> Variant of wt sequence (Seq2)

<400> 46
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Glu Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Arg Leu His Gly Pro Glu Val Leu Ala Ile Ile Ala Glu Thr
        195                 200                 205
Ile Gln Met Ser Tyr Ser Asn Asn Asn Pro Leu Ser Glu Val Ala Phe
    210                 215                 220
Gly His Ala Lys Thr Ala Ala Glu Val Ala Ala Leu Met Pro Leu Leu
225                 230                 235                 240
Thr Ala Asn Tyr Asp Phe Thr Asn Asp Leu Pro Tyr Val Ala Arg Arg
                245                 250                 255
Gly Ala Ser Val Leu Met Asn Gln Ile Ala Leu Ala Leu Asn Thr Glu
            260                 265                 270
His Gln Ala Ala Ala Ala Gln Ala Asp Ala Pro Pro Asp Ala Lys Trp
        275                 280                 285
Leu Leu Tyr Val Ala His Asp Thr Asn Ile Ala Lys Leu Arg Thr Met
    290                 295                 300
Leu Gly Phe Thr Trp Gln Met Gly Asp Tyr Pro Arg Gly Asn Ile Pro
305                 310                 315                 320
Pro Ala Gly Ser Leu Ile Phe Glu Arg Trp Arg Asn Gln Gln Ser Gly

325                 330                 335
Gln Arg Val Val Arg Ile Tyr Phe Gln Ala Gln Ser Leu Asp Gln Ile
            340                 345                 350
Arg Gly Leu Ala Ala Leu Asp Asp Ser His Pro Pro Leu Arg Ser Glu
        355                 360                 365
Phe Ser Met Pro Asp Cys Gln Lys Thr Asp Ile Gly Thr Leu Cys Pro
    370                 375                 380
Tyr Asp Ala Val Met Lys Arg Leu Asn Asp Ala Val Asp Arg Thr Ala
385                 390                 395                 400
Leu Leu Pro Val His Tyr Thr Pro His His His His His His
                405                 410

<210> 47
<211> 414
<212> PRT
<213> Artificial Sequence

<220>
<223> Variant of wt sequence (Seq2)

<400> 47
Asp Thr Pro Ser Ser Gly Trp Thr Leu Glu Lys Val Val Glu Val Ser
1               5                   10                  15
Arg His Gly Val Arg Pro Pro Thr Glu Ser Asn Val Lys Thr Ile Gln
            20                  25                  30
Glu Gly Thr Asp Arg Gln Trp Pro Thr Trp Leu Thr Gln Tyr Gly Glu
        35                  40                  45
Leu Thr Gly His Gly Tyr Ala Ala Ala Glu Leu Lys Gly His Tyr Glu
    50                  55                  60
Gly Gln Tyr Leu Arg Gln His Gly Leu Leu Cys Gln Ala Cys Pro Ala
65                  70                  75                  80
Pro Gly Asp Val Phe Val Trp Ala Ser Pro Leu Gln Arg Thr Arg Glu
                85                  90                  95
Thr Ala Met Ala Leu Met Asp Gly Val Phe Pro Gly Cys Gly Val Ala
            100                 105                 110
Ile Gln Gly Pro Glu Asp Glu Asp Asn Asp Pro Leu Phe His Ala Glu
        115                 120                 125
Thr Ala Gly Val Thr Leu Asp Gln Gln Gln Val Lys Ala Asp Leu Gln
    130                 135                 140
Asn Ala Met Gln Asn Lys Ser Ala Ala Gln Ile Gln Ala Gln Trp Gln
145                 150                 155                 160
Pro Ala Ile Asp Arg Leu Lys Gln Ala Val Cys Leu Pro Asp Lys Pro
                165                 170                 175
Cys Pro Ala Phe Ser Thr Pro Trp Glu Val Lys Glu Asp Asn Asn Gly
            180                 185                 190
Ser Ile Arg Leu His Gly Pro Glu Val Ala Ala Ala Glu Val Leu Ala
        195                 200                 205
Ile Ile Ala Glu Thr Ile Gln Met Ser Tyr Ser Asn Asn Asn Pro Leu
    210                 215                 220
Ser Glu Val Ala Phe Gly His Ala Lys Thr Ala Ala Glu Val Ala Ala
225                 230                 235                 240
Leu Met Pro Leu Leu Thr Ala Asn Tyr Asp Phe Thr Asn Asp Leu Pro
                245                 250                 255
Tyr Val Ala Arg Arg Gly Ala Ser Val Leu Met Asn Gln Ile Ala Leu
            260                 265                 270
Ala Leu Asn Thr Glu His Gln Ala Asp Ala Pro Pro Asp Ala Lys Trp
        275                 280                 285
Leu Leu Tyr Val Ala His Asp Thr Asn Ile Ala Lys Leu Arg Thr Met
    290                 295                 300
Leu Gly Phe Thr Trp Gln Met Gly Asp Tyr Pro Arg Gly Asn Ile Pro
305                 310                 315                 320
Pro Ala Gly Ser Leu Ile Phe Glu Arg Trp Arg Asn Gln Gln Ser Gly

                325                 330                 335
Gln Arg Val Val Arg Ile Tyr Phe Gln Ala Gln Ser Leu Asp Gln Ile
            340                 345                 350
Arg Gly Leu Ala Ala Leu Asp Asp Ser His Pro Pro Leu Arg Ser Glu
        355                 360                 365
Phe Ser Met Pro Asp Cys Gln Lys Thr Asp Ile Gly Thr Leu Cys Pro
    370                 375                 380
Tyr Asp Ala Val Met Lys Arg Leu Asn Asp Ala Val Asp Arg Thr Ala
385                 390                 395                 400
Leu Leu Pro Val His Tyr Thr Pro His His His His His His
                405                 410